# EUROPEAN PATENT SPECIFICATION

(11) **EP 1 572 182 B1**
(45) Date of publication and mention of the grant of the patent: **31.01.2007**
(21) Application number: 03770922.7
(22) Date of filing: 11.11.2003
(51) Int. Cl.: A61K 31/195, A61K 36/00, A61P 1/00

(54) **COMPOSITION FOR THE TREATMENT OF GASTROINTESTINAL DISORDERS**
ZUSAMMENSETZUNG ZUR BEHANDLUNG VON GASTROINTESTINALEN BESCHWERDEN
COMPOSITIONS POUR LE TRAITEMENT DE TROUBLES GASTRO-INTESTINAUX

(30) Priority: 11.11.2002 DK 200201736
(43) Date of publication of application: 14.09.2005
(73) Proprietor: Pharmalett A/S, 6000 Kolding (DK)
(72) Inventor: AGGER, Nicolai, DK-5800 Nyborg (DK)
(74) Representative: Larsen, Hans Ole
(86) International application number: PCT/DK2003/000773
(87) International publication number: WO 2004/043451

(56) References cited:
- EP-A- 0 382 306
- WO-A-82/02650
- WO-A-85/01441
- US-A- 3 898 328
- US-A- 4 164 568
- US-A- 6 007 808
- US-A- 6 066 341
- DATABASE WPI Section Ch, Week 199309 Derwent Publications Ltd., London, GB; Class B05, AN 1993-069507 XP002269306 & HU 61 474 A (PHYLAXIA OLTOANYAGTERMELOE) , 28 January 1993 (1993-01-28)

## Description

The invention relates
- to a preparation comprising 5-50% by weight of Isphagula Husk; 1-20% by weight of at least one amino acid; 20-80% by weight of at least one carbohydrate and electrolytes for use as a therapeutical agent,
- and further to a preparation containing Isphagula Husk; at least one amino acid; and at least one carbohydrate and electrolytes said Isphagula Husk, at least one amino acid and at least one carbohydrate and electrolytes as a combined preparation for simultaneous or sequential use in treating a state of disorder of the intestinal system of monogastric animals, including human beings,
- and further to a preparation for treating a state of disorder of the intestinal system of monogastric animals, including human beings, said preparation comprising 5-50% by weight of Isphagula Husk; 1-20% by weight of at least one amino acid; 20-80% by weight of at least one carbohydrate and electrolytes,
- and further to a preparation for restoring the epithelium layer of the intestines of mammals, including human beings, said preparation comprising 5-50% by weight of an agent comprising Isphagula Husk; 1-20% by weight of at least one amino acid; 20-80% by weight of at least one carbohydrate and electrolytes.

A state of disorder of the intestinal system is in particular all intestinal disorders in which the epithelial layer is damaged, mostly as a result of malabsorption diarrhoea, also associated with dehydration.

### BACKGROUND OF THE INVENTION

Every year, the enteric pathogens are responsible for many neonatal calf deaths resulting in several $100 million in losses to the agricultural economy of the world. Young calves are stressed by transportation, a change in environment and diet when weaned. Stress comes in many forms, however, the foremost effect of stress on the gastrointestinal tract is to decrease mucocal blood flow and thereby compromise the integrity of the mucosal barrier. An important part of the barrier function is to prevent transit of bacteria from the lumen through the epithelium. Stress may lead to development of diseases such as diarrhoea, also known as scours, and is associated with the disruption of the gastrointestinal barrier in conjunction with a dehydration of the young animal. Local infections by bacteria and virus, exposure to toxins, physical insults and many systemic diseases lead to the disruption. The disruption may be mild and relatively easy to recover or it may be fatal. The structural features of the intestinal barrier, such as the villi which are part of the mucosa, can be totally destroyed and absent during the time of these states of disorder of the intestinal system. For example corona virus (Cryptosporidium parvum) and rota virus all cause destruction of the mature cells on the tips of the intestinal villus. Absorption of electrolytes and simple sugars is drastically lowered during such disorders, and therefore the young animal suffers from the inability to absorb the nutrients and the water which it may consumes.

### DESCRIPTION OF THE BACKGROUND ART

EP 0 160 015 describes a preparation for rehydrating monogastric animals, including human beings, and new-born ruminants suffering from diarrhoea, comprising an absorbent intumescent agent, e.g. Isphagula Husk, electrolytes, glucose and lactose-decomposing enzyme(s). The preparation is especially suited for the treatment of non-infectious diarrhoea and diarrhoea caused by rota and corona viruses. It is mentioned that Isphagula Husk shows a considerable ability to decompose lactose, and that experiments indicate that mucous produced by Isphagula Husk replaces the damaged mucous layer in the subjects suffering from intestinal infections in such a way that it acts as a bioadhesive polymer which may enchance glucose absorption. Furthermore, the preparation is described as having inter alia a protective effect on the intestinal mucosa.

EP 0 474 282 describes the use of polysaccharide-containing materials in preparations for wound treatment. The material used is Isphagula Husk (or psyllium), described as a fibre material. When used in the field of wound treatment, the Isphagula Husk is, among other characteristics, found to have the advantage that a non-specific binding occurs between the mucopolysaccharides and the cell walls of the bacteria present in the wound. Furthermore, the fibres are able to absorb moisture or to bind wound moisture, and to produce a mucous material in combination with the moisture. The fibre materials have successfully been used in combination with other active substances, which can be growth stimulators and electrolytes among others. The Isphagula Husk is not described as enhancing cell growth in the description of the wound treatment. The preparations are preferably in the form of porous holder, for example a sachet or a compress-like product, which comes in contact with the skin. The preparations can also be used in inter alia the epithelialisation stage, as a moist medium is formed due to gel formation taking place between the underside of the sachet, the cushion and the wound surface.

US A 6 066 341 describes composition for treating diarrhoea in animals such as calves, a composition which includes psyllium. Psyllium is the major constituent of the composition and is present at about 65-70 percent by weight thereof.
WO 85/01441 A also describes a preparation against diarrhoea in animals such as calves, which preparation applies psyllium in an amount of 40-43 percentage by weight thereof.

In both these examples the preparations contain relatively large amounts of Isphagula Husk, which carries the risk of side effects, especially constipation in the animals.

In "New therapies for calf diarrhoea: Therapy and prevention for the new millennium", *Elaine Hunt, North Carolina State University College of Veterinary Medicine,* it is mentioned that glutamine may play a role in diarrhoeal disease through several mechanisms, and that it plays a role in maintaining mucosal integrity of the gut, and in controlled situations also has been successful in increasing the recovery of the damaged intestinal mucosa. It has been found that glutamine has a direct effect on the absorption of solute in the calf intestine and glutamine is proposed to be a significant component of electrolyte solutions in the future for treating calf diarrhoea. Glutamine, in addition to sparing the intestinal mucosa during nutrient deprivation, has been found to increase the speed of recovery of damaged mucosa. When glutamine was combined with TGF alpha it stimulated recovery from ischemia/reperfusion injury and did the same in porcine rotavirus enteritis. Furthermore, it has been shown that glutamine stimulates glutamine-coupled Na⁺ absorption in the cells on the tips of the intestinal villus in a pig, and that glutamine stimulates water and electrolyte absorption much more than glucose. It is furthermore found that a glutamine transporter is present throughout the ileal villus in the calf, and therefore glutamine based oral electrolyte solutions should be more effective than glucose based solutions in osmotic diarrhoeas in calves. Other substances besides glutamine are mentioned that may stimulate local proliferation of cells in the process of mucosal repair, such as arginine, prostaglandins and certain growth factors, which together with glutamine have been found to stimulate mucosal repair in in vitro studies.

US patent no. 4,711,780 discloses a medication for treating the surface epithelium comprising vitamin C, a zinc salt and a sulphur amino acid, which medication may also comprise a mucopolysaccharide. The mucopolysaccharide is described as acting as a barrier preventing toxins on the skin surface from penetrating into the blood circulation system, which otherwise leads to septicaemia. The amount of mucopolysaccharide, which could be extracted from the aloe vera plant, is present in amount from about 0.05 to 10 % by weight. No mechanism is disclosed for the co-action of mucopolysaccharide in assisting the cell growth and in the uptake of amino acids.

In "Potential benefits of Psyllium mucilloid supplementation of oral replacement formulas for neonatal calf scours", *Continuing Education Article 7, North American Edition vol. 14, no. 2, February 1992 247 by Martin J. Fettman,* it is stated that intestinal cell proliferation and function may benefit from fibre-enhanced rates of short-chain fatty acid production. Addition of psyllium to the colonocyte cultures from humans at high risk of developing colon cancer has also been found to stimulate microbial metabolism, short-chain fatty production and subsequently colonocyte growth. Oral replacement formulas comprising psyllium mucilloid may, among other things, have stimulatory effects on the enterocyte proliferation, which may be beneficial in the recovery form enteritis and subsequent return to normal dietary intake. Furthermore, it is mentioned that an experiment was performed where cellulose was added to an elemental liquid diet in rats, and small intestinal cell renewal rates were increased significantly.

In "Evaluation of a glutamine-containing oral rehydration solution for the treatment of calf diarrhoea using an "Escherichia coli" model", *Brooks et al, The Veterinary Journal 1997, 153, 163-170,* several different compositions of ORS's (oral rehydration solutions) have been investigated for there beneficial effects on blood glucose and body weight for calves which had E. coli administered orally. A single amino acid is focused on, namely glutamine, which is described as having the potential to promote enteric sodium uptake, and being important in sustaining the villus form and function (referring to "Glutamine and preservation of gut integrity", Van der Hulst et al., Lancet 341, 1363-5 (1993)), and possibly also as supporting the integrity barrier and immune function in the intestine (referring to "Intestinal fuels: glutamine, short-chain fatty acids and dietary fiber", Evans et al., Journ. Parenteral and Enteral Nutrition, 17, 47-55 (1992)). The results are consistent with a previous result: the ability of glutamine to stimulate both neutral and electrogenic sodium absorption. It is mentioned that one of the beneficial properties of glutamine is to sustain the effect of epidermal growth factor on intestinal mucosal cell proliferation (referring to "Glutamine is essential for epidermal growth factor-stimulated intestinal cell proliferation, Ko et al., Surgery, 114, 147-54 (1993)).

In "Effect of glutamine or glycine containing oral electrolyte solutions on mucosal morphology, clinical and biochemical findings, in calves with viral induced diarrhea", *J.M. Naylor et al*, *Can J Vet Res 1997*; *61: 43*-*48*, calves were treated with 1 to 3 oral electrolytes each with a single amino acid: 40 mmol/l glycine or 40 mmol/l glutamine or 400 mmol/l glutamine. The type of electrolyte significantly affected duodenal villus height. There was no significant difference in small intestinal surface area between groups. Overall, the trial did not suggest that substituting glutamine for glycine in oral electrolyte solutions improves treatment of diarrheic calves or speeds of mucosal healing.

### DISCLOSURE OF THE INVENTION

It is an object of the invention to provide an improved preparation for the treatment of monogastric animals, including human beings, suffering from diarrhoea, in particular to provide an improved healing of the mucosa during diarrhoea.

It is an object of the present invention to provide an improved preparation for the restoration of the epithelium layer of the intestines.

It is an object of the present invention to provide an improved preparation for supplying nutrients to the epithelium layer of the intestines in an improved way.

It is an object of the present invention to provide an improved preparation for supplying nutrients to a monogastric animal, including human beings, suffering from diarrhoea, in particular a young animal.

It is an object of the present invention to provide a preparation for enhanced cell growth, especially for the cells of the intestinal epithelium.
It is an object of the present invention to provide a preparation for use as a therapeutical agent.

These objects are achieved by the following preparations.

In one embodiment the preparation comprises 5-30% by weight of Isphagula Husk, and 1-20% by weight of at least one amino acid, and 20-80% by weight of at least one carbohydrate and electrolytes for use as a therapeutical agent.

In one embodiment the preparation comprises 5-30% by weight of Isphagula Husk, and 1-20% by weight of at least one amino acid, and 20-80% by weight of at least one carbohydrate and electrolytes, said Isphagula Husk, at least one amino acid and at least one carbohydrate and electrolytes as a combined preparation for simultaneous or sequential use in treating a state of disorder of the intestinal system of monogastric animals, including human beings.

In one embodiment the preparation for treating a state of disorder of the intestinal system of monogastric animals, including human beings, comprises 5-30% by weight of Isphagula Husk, and 1-20% by weight of at least one amino acid, and 20-80% by weight of at least one carbohydrate and electrolytes.

In one embodiment the preparation for restoring the epithelium layer of the intestines of mammals, including human beings, comprises 5-30% by weight of Isphagula Husk, and 1-20% by weight of at least one amino acid, 20-80% by weight of at least one carbohydrate and electrolytes.

The above-mentioned preparations may consist of the stated ingredients, exclusively.

The diarrhoea may be all kinds of diarrhoea, especially non-infectious diarrhoea, malabsorption/maldigestive, osmotic diarrhoea for example caused by Rotavirus and Corona "Cryptosporidium parvum" viruses which viruses all cause destruction of the mature cells on the tips of the intestinal villus. The preparation according to the invention is suited for the treatment of any diarrhoea associated with a need for supplying nutrients to the cells in the intestinal.

In one embodiment the amount of Isphagula Husk is in the interval of 10-30% by weight, preferably 15-30% by weight, more preferably 25-30% by weight.

Isphagula Husk is a fibrous material, which is also termed psyllium (husk), and is described in EP 0 160 015 and EP 0 474 282, which are hereby incorporated by reference. The fibre material to be used according to the invention comprises a so-called mucopolysaccharide originating from Plantago ovata containing a polyxylose basic structure and one or more side chains chosen from the group comprising galacturonic acid, galactose, mannose, glucose, fucose, ramnose and arabinose. Isphagula Husk may be characterised as an intumescent agent, absorbing water, and shows a considerable ability to decompose lactose, and furthermore to provide a non-specific binding between the mucopolysaccharides and the cell walls of bacteria.

In one embodiment the amount of the at least one amino acid is in the interval of 1-12% by weight, preferably 2-9 % by weight, more preferably 3-7 % by weight.

In one embodiment the amount of carbohydrate is in the interval of 25-50% by weight, preferably 30-45% by weight, more preferably 35-40% by weight.

The carbohydrate is defined as being a simple sugar such as a monosaccharide, preferably glucose as dextrose monohydrate, or it may be a disaccharide such as sucrose. Glucose is absorbed in the small intestine of the animal and provides a source of energy and aids the recovery process. The relative amounts of the carbohydrate and Isphagula Husk in the preparation according to the invention is such that, when the preparation is administered to the animal, the preparation contains an amount of the total added carbohydrate which is not bound to Isphagula Husk, but is present in the liquid phase of the preparation mixture without a binding to Isphagula Husk.

In one embodiment the amount of electrolyte is in the interval of 8-40% by weight, preferably 12-30% by weight, more preferably 15-25% by weight.

A mixture of amino acids according to the invention may be provided from lactic yeast ("Milchhefe") that has been processed to provide the soluble components of the yeast cells. The process may be either, or a combination of, the following processes: plasmolysis, autolysis, thermolysis or mechanical disruption. The processed lactic yeast is preferably supplemented with glutamine. The lactic yeast mixture is preferred as a source of amino acids due to a relatively high content of amino acids in an inexpensive and readily available product. The indicative value of the content of amino acids in a lactic yeast mixture is 48-52% by weight. In theory, any cells which may be subjected to the processes mentioned above may be processed and used as a source of amino acids.

The lactic yeast cells are preferred due to the fact that the composition of amino acids resembles the composition found in colostrum. Also, the composition of vitamins, minerals, sterols, polyunsaturated fatty acids, gluathion resembles that of colostrum. This amino acid composition is well suited for treating a young animal suffering from a state of disorder of the intestinal system, in particular a calf, a newborn calf or a piglet.
In one embodiment of the invention the at least one amino acid is comprised in a yeast extract, where the yeast is lactic yeast.

In one embodiment of the invention the at least one amino acid is comprised in the soluble components of lactic yeast.

In one embodiment of the invention the at least one amino acid is comprised in plasmolysed, dried lactic yeast.

In one embodiment of the invention the at least one amino acid is comprised in a dry extract of selected lactic yeast.

The extract of lactic yeast may also be a liquid yeast extract (with a dry solids content of 50 to 65%) or a highly viscous paste type (with a dry solids contents of 70 to 80%).

In one embodiment the preparation comprises at least one amino acid selected from the group consisting of all known amino acids, preferably at least one amino acid selected from the group consisting of glutamine, arginine, lysine, histidine, phenylalanine, tyrosine, leucine, isoleucine, methionine, valine, alanine, glycine, proline, glutamic acid, serine, threonine, aspartic acid, tryptophan, cystine, more preferably at least one amino acid selected from the group consisting of glutamine, arginine, alanine and glycine. Glutamine, arginine, alanine and glycine are thought to be of particular value to the restoration of the epithelium.

In one embodiment the mixture of amino acids is characterized by having a relative amount of amino acids, which may be found in the colostrum of mammals, preferably in the colostrum of a bovine animal.

In one embodiment the mixture of amino acids may be a kit of parts of a source of proteins and suitable protease for the controlled degradation of the proteins into said mixture.

In one embodiment the amount of glutamine may be in the interval of up to 10% by weight, preferably up to 5% by weight, more preferably 0.1-4% by weight, even more preferably 0.2-3%.

The glutamine is L-glutamine and the source may be L-glutamine itself or L-alanyl-L-glutamine (known as the trademark "Glutamax I") or glycyl-L-glutamine (known as the trademark "Glutamax II"). Glutamine is important in the mucosal regenerative processes. Glutamine has been found to spare the integrity of the gut mucosa in nutrient deprivation states in many species. By isolating segments of calf ileum it has been demonstrated that glutamine will function to maintain the integrity of the gut mucosa which has otherwise been deprived of local nutrients. It has also been found to increase the speed of recovery of damaged mucosa.

In one embodiment the amount of arginine is in the interval of up to 5% by weight, preferably up to 3% by weight, more preferably 0.1-2% by weight, even more preferably 0.1-0.5%.

In one embodiment the preparation comprises at least one of the salts comprised by the electrolytes and is at least one of the salts that will replace at least one of the salts lost by diarrhoea. When administering a preparation according to the invention the salts lost by diarrhoea are provided by replaced salts comprised by the electrolytes in order to bring about both rehydration or stop dehydration.

In one embodiment said at least one carbohydrate is glucose.

In one embodiment the preparation comprises electrolytes which are a mixture of at least two of the substances selected from the group consisting of magnesium oxide, magnesium carbonate hydroxide, magnesium hydroxide, magnesium silicate, calcium silicate, calcium carbonate, sodium chloride, potassium chloride, sodium hydrogen carbonate, potassium hydrogen carbonate, aluminium phosphate, aluminium hydroxide, citric acid, sodium citrate, trisodium citrate dihydrate and potassium citrate.

In one embodiment the preparation comprises electrolytes which are a mixture of at least two of the substances selected from the group consisting of magnesium hydroxide, sodium chloride, potassium chloride, sodium hydrogen carbonate, citric acid, trisodium citrate dihydrate and sodium citrate.

In one embodiment the preparation comprises at least one filler, at least one taste corrigent, at least one colouring agent.

In one embodiment the filler is a fibrous bran material.

In one embodiment the filler is wheat flour.

In one embodiment the preparation comprises a pharmaceutically acceptable colouring agent.

In one embodiment the preparation comprises the colouring agent FD&C RED #40.

In one embodiment the preparation comprises alfa-tocoferol (natural vitamin E).

In one embodiment the preparation is composed of 27.16% Isphagula Husk, 10.66% of lactic yeast including glutamine, 19.75% of electrolytes which are made up of 3.30% potassium chloride, 7.08% sodium hydrogen carbonate, 4.85% sodium chloride, 3.45% trisodium citrate dihydrate, 1.07% magnesium hydroxide; 38.10% dextrose monohydrate, 0.87% nicotinamide, 0.30% flavouring agent, 0.20% silicium dioxide, 2.43% wheat flour, 0.03% feed colouring agent, 0.50% alfa-tocoferol (natural vitamin E), where the percent by weight is calculated on the basis of the finished preparation.

In one embodiment the preparation is used for the manufacture of a medicament for treating diarrhoea.

In one embodiment the preparation is used for the manufacture of a medicament for treating the epithelium layer, preferably the epithelium layer of the intestinal, more preferably the epithelium cells of the villi.

Stress is almost always associated with mucosal erosions. The villi, which are a structural part of the mucosal barrier, may be disrupted to a lesser or larger extent during a state of disorder of the intestinal system such as during diarrhoea. The health condition is worsened dramatically upon such disruption, as the absorption of nutrients in the intestinals does not function properly. Until healing of the villi has completed to an extent where absorption of substances has a positive effect on the overall condition of the animal, the animal will be in critical state. It is therefore crucial for the recovery to be expedient, often a few hours are thought to be important. Healing requires that the epithelial cells on the margins of the defect proliferate, differentiate and migrate into the damaged area to restore the normal cellular architecture and function. The process of healing may be described as a process with positive feedback with the growing villi being gradually able to absorb more and more nutrient substances. A small positive effect in the early stage is thus believed to have a great effect on the overall time of recovery and hence on the chances of survival.
It has quite unexpectedly been found that the addition of an agent comprising a rehydrant containing Isphagula Husk and a mixture comprising amino acids to a cell culture enhances cell growth significantly compared to adding either the rehydrant containing Isphagula Husk alone to the cell culture or a rehydrant without Isphagula Husk and the mixture to the cell culture.

A possible mechanism for the unexpected enhanced positive effect on cell growth shown below for an agent with a combination of Isphagula Husk and an amino acid mixture could be that Isphagula Husk acts as a *mediator.* A mediator provides a potential enhanced means for communication from the exterior of the cell to the inside of a cell. Isphagula Husk cannot pass the cell wall itself due to its large structure and has no direct effect on cell growth. As observations nevertheless show an effect of Isphagula Husk in combination with a mixture of amino acids, it might well be that Isphagula Husk is present at the cell wall level where it aids the uptake of amino acids. To our knowledge, among the many characteristics ascribed to Isphagula Husk the characteristic of Isphagula Husk to act as a mediator has not been described before.

Without a *mediator* for enhanced communication with the interior of the cell, nutrients will not be supplied so efficiently, comparatively, and this may explain why there was only a modest positive effect (see below) on the growth of a cell culture in the presence of a mixture comprising an amino acid and a rehydrant without Isphagula Husk.

The agent comprising a combination of a rehydrant containing Isphagula Husk and a mixture comprising amino acids will be used for the treatment of diarrhoea among all offspring of ruminants as long as these are monogastric, and for the treatment of non-infectious diarrhoea and diarrhoea caused by rota and corona viruses among all other one-stomached animals, including humans.

The above-mentioned findings are presented below in more detail.

The cell proliferation in a standard cell culture medium was examined in the presence and in the absence of Isphagula Husk.

A standard cell culture medium was used, Dulbecco's Modified Eagle's Medium (DMEM), with or without glutamine as indicated below.

### Two solutions were prepared:

Cell culture kit I contained DMEM with glutamine, 10% fetal calf serum, 100 IU/ml penicillum and 100 IU/ml streptomycin.
Cell culture kit II contained DMEM with glutamine, 10% fetal calf serum, 100 IU/ml penicillum and 100 IU/ml streptomycin plus 20% Isphagula Husk.

The cells used were provided from human foreskins obtained from the surgical department of the Academic Medical Centre, Amsterdam. The epidermis was removed; the dermal layer was cut into fine pieces and incubated in 0.25% dispase/0.25% collagenese for 2 h at 37 °C. The suspension was filtered through an infusion chamber; the cells were centrifuged and resuspended in culture medium. The fibroblast cultures were maintained at 37 °C in air and 5% CO₂.

Per solution, three well culture dishes were prepared. Fibroblasts were seeded at a density of 10³ cells per square cm. To quantify cell growth, the number of cells was determined by measuring the lactate dehydrogenase (LDH) activity as described by Matsuda et al. (automated sampler, Cobas Fara, Hoffman LaRoche Ltd., Basel, Switzerland) at day 0, day 1, day 3, day 7 and day 14 and given in arbitrary units (a.u.). Table 1 shows the raw data of the LDH results.

**Table 1**

| LDH levels measured for solutions I and II. | | |
|---|---|---|
| | Solution | |
| | I | II |
| Day 0 | 165, 160, 162 | 171 |
| **Day 0 (mean)** | **162** | **171** |
| Day 0 (standard deviation) | 2.5 | - |
| Day 1 | 201, 210, 203 | 221 |
| **Day 1 (mean)** | **205** | **221** |
| Day 1 (standard deviation) | 4.7 | - |
| Day 3 | 405,413,410 | 510 |
| **Day 3 (mean)** | **409** | **510** |
| Day 3 (standard deviation) | 4.0 | - |
| Day 7 | 824, 870, 802 | 971 |
| **Day 7 (mean)** | **832** | **971** |
| Day 7 (standard deviation) | 34.7 | - |
| Day 14 | 999, 1005, 1201 | 1041 |
| **Day 14 (mean)** | **1068** | **1041** |
| Day 14 (standard deviation) | 114.9 | - |

The differences between I and II for the LDH activity indicate that the addition of Isphagula Husk enhances cell growth.

The cell proliferation in a standard cell culture medium was also examined in the presence of rehydrants of different compositions.

Five solutions III, IV, V, VI and VII were prepared (all stated percentages are by weight):
Cell culture kit III contained:
   20% DMEM with glutamine, 100 IU/ml penicillum and 100 IU/ml streptomycin;
   60% rehydrant no. 1 (see table below for the composition);
   20% Protibel.
Cell culture kit IV contained:
   20% DMEM without glutamine, 100 IU/ml penicillum and 100 IU/ml streptomycin;
   80% rehydrant no. 1.
Cell culture kit V contained:
   20% DMEM without glutamine, 100 IU/ml penicillum and 100 IU/ml streptomycin;
   80% of a standard WHO rehydrant (29.8 g glucose, 1.9 g KCI, 2.1 g NaHCO₃, 0.6 g NaCl/litre).
Cell culture kit VI contained:
   20% DMEM without glutamine, 100 IU/ml penicillum and 100 IU/ml streptomycin;
   80% of a hypertonic rehydrant (90 g glucose, 1.9 g KCI, 2.1 g NaHCO₃, 0.6 g NaCl/litre).
Cell culture kit VII contained:
   20% DMEM with glutamine, 100 IU/ml penicillum and 100 IU/ml streptomycin;
   60% rehydrant no. 1, but depleted of Isphagula Husk; 20% Protibel.

**Composition of Rehydrant no. 1**

| | |
|---|---|
| Ca²⁺ | 4 mmol/l |
| Na⁺ | 78 mmol/l |
| K⁺ | 37 mmol/l |
| Cl⁻ | 71 mmol/l |
| Bicarbonate (HCO₃⁻), and propianate (CH₃CH₂COO⁻), and phosphate (PO₄³⁻), total: | 48 mmol/l |
| Isphagula Husk | 21% |
| Protibel | 20% |

**Composition of Protibel ("lactic yeast", "Mitchhefe").**

| Data taken from manufacturer's analytic characteristics, Protibel D100P, Bel Industries. | | |
|---|---|---|
| Component | Indicative | Unit |
| | value | |
| H₂O | 6.3 | % |
| Ashes | 6.2 | % |
| Fat (very similar to that of milk with a high proportion of essential polyunsaturated fatty acids) | 6.6 | % |
| Total sugar (mainly fructose, glucose, galactose) | 22.7 | % |

| Amino acids | | |
|---|---|---|
| Arginine | 2.3 | g/100 g dry yeast |
| Lysine | 3.2 | g/100 g dry yeast |
| Histidine | 0.85 | g/100 g dry yeast |
| Phenylalanine | 1.88 | g/100 g dry yeast |
| Tyrosine | 1.43 | g/100 g dry yeast |
| Leucine | 3.23 | g/100 g dry yeast |
| Isoleucine | 2.08 | g/100 g dry yeast |
| Methionine | 0.57 | g/100 g dry yeast |
| Valine | 2.36 | g/100 g dry yeast |
| Alanine | 2.53 | g/100 g dry yeast |
| Glycine | 2.07 | g/100 g dry yeast |
| Proline | 1.59 | g/100 g dry yeast |
| Glutamic acid | 6.78 | g/100 g dry yeast |
| Serine | 2.12 | g/100 g dry yeast |
| Threonine | 2.16 | g/100 g dry yeast |
| Aspartic acid | 4.07 | g/100 g dry yeast |
| Tryptophan | 0.56 | g/100 g dry yeast |
| Cystine | 0.39 | g/100 g dry yeast |
| Other amino acids | 4.32 | mg/100 g dry yeast |
| Glutathione | 0.2 | % |
| Subtotal of proteins | 48-52 | % |

| Vitamins (in selection) | | |
|---|---|---|
| B1 | 0.96 | mg/100 g dry yeast |
| B2 | 4.49 | mg/100 g dry yeast |
| B6 | 0.99 | mg/100 g dry yeast |
| B12 | 0.00653 | mg/100 g dry yeast |
| C | 3.67 | *mg*/*100 g dry yeast* |
| Minerals | 6.5-8.5 | % |
| Free sterols | 0.2 | % of neutral lipids |

The cells used were provided from human foreskins obtained from the surgical department of the Academic Medical Centre, Amsterdam. The epidermis was removed; the dermal layer was cut into fine pieces and incubated in 0.25% dispase/0.25% collagenese for 2 h at 37 °C. The suspension was filtered through an infusion chamber; the cells were centrifuged and resuspended in culture medium. The fibroblast cultures were maintained at 37 °C in air and 5% CO₂.

Per solution, three well culture dishes were prepared. Fibroblasts were seeded at a density of 10³ cells per square cm. The number of cells was determined by measuring the lactate dehydrogenase (LDH) activity as described by Matsuda et al. (automated sampler, Cobas Fara, Hoffman LaRoche Ltd., Basel Switzerland) at day 0, day 1, day 3, day 7 and day 14, and the measurement results were used to quantify cell growth. Table 2 shows the raw data of the LDH results.

Table 2 shows the raw data.

**Table 2**

| LDH levels measured for solutions III, IV, V, VI and VII. | | | | | |
|---|---|---|---|---|---|
| | Solutions | | | | |
| | III | IV | V | VI | VII |
| Day 0 | 170, 163, 162 | 171, 155, 164 | 180, 161, 170 | 171, 166, 162 | 165 |
| **Day 0 (mean)** | **165** | **163** | **170** | **166** | **165** |
| Day 0 (standard deviation) | 4.4 | 8.0 | 9.5 | 4.5 | - |
| Day 1 | 174, 170, 180 | 140, 138, 130 | 139, 130, 128 | 101, 90, 88 | 168 |
| **Day 1 (mean)** | **175** | **136** | **132** | **93** | **168** |
| Day 1 (standard deviation) | 5.0 | 5.3 | 5.9 | 7.0 | - |
| Day 3 | 285, 270,314 | 175, 167,180 | 167, 157, 150 | 18, 7, 12 | 200 |
| **Day 3 (mean)** | **290** | **174** | **158** | **12.3** | **200** |
| Day 3 (standard deviation) | 22.4 | 6.6 | 8.5 | 5.5 | - |
| Day 7 | 421, 447,437 | 166, 150, 146 | 151, 143, 128 | 0,0,0 | 291 |
| **Day 7 (mean)** | **435** | **154** | **141** | **0** | **291** |
| Day 7 (standard deviation) | 13.1 | 10.6 | 11.7 | - | - |
| Day 14 | 593, 621,600 | 136, 144, 129 | 118, 112, 108 | 0, 0, 0 | 395 |
| **Day 14 (mean)** | **605** | **136.6** | **113** | **0** | **395** |
| Day 14 (standard deviation) | 14.6 | 7.5 | 5.0 | - | - |

Figure 1 shows the graphical representation of the results. The results from Example 1 are also included in fig. 1. The difference between the results is statistically significant (p<0.05) with the exception of the results of solutions II and VII.

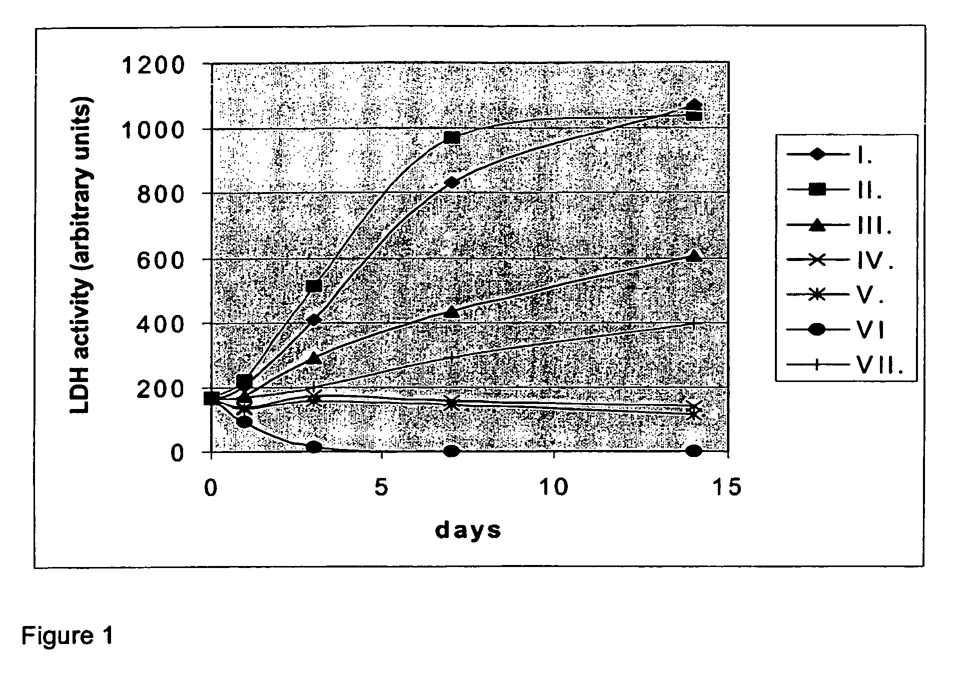

By comparing the experimental results shown in figure 1 the positive effect on the LDH level of solution III comprising a combination of a rehydrant containing Isphagula Husk and a mixture comprising amino acids is seen. The positive effect is seen by comparison of the LDH level of the solutions IV and V, both comprising said rehydrant containing Isphagula Husk, but without the mixture comprising amino acids, with the LDH level of solution VII comprising a combination of the mixture comprising amino acids and a rehydrant without Isphagula Husk.

Also, by comparing the experimental results shown in figure 1 the positive effect on the LDH level of solution II comprising a combination of a standard cell culture kit containing fetal calf serum and Isphagula Husk is seen. The positive effect is seen by comparison of the LDH level of solution I comprising the standard cell culture kit containing fetal calf serum alone, without Isphagula Husk.

### Example 1

The preparation according to the invention may e.g. be composed as follows:

| | |
|---|---|
| Dextrose monohydrate | 38.10% |
| Psyllium powder (Isphagula Husk) | 27.16% |
| Potassium chloride, KCI | 3.30% |
| Sodium Hydrogen Carbonate | 7.08% |
| Sodium Chloride, NaCl | 4.85% |
| Trisodium citrate dihydrate | 3.45% |
| Nicotinamide | 0.87% |
| Lactic yeast mixture with high contents of proteins, B-vitamins and ascorbic acid including glutamine | 10.66% |
| Flavouring agent (sweet peach) | 0.30% |
| Silicium dioxide | 0.20% |
| Wheat flour | 2.43% |
| FD&C RED #40 (feed colouring agent) | 0.03% |
| Magnesium hydroxide, MgOH | 1.07% |
| alfa-tocoferol (natural vitamin E) 60% | 0.50% |
| TOTAL | 100.00% |

Percentages are stated as percentages by weight.

The individual ingredients are all available as dry powders and are mixed mechanically. The preparation according to the invention may not be administered in dry form, but must be suspended in water and administered as a solution or suspension, as described in EP 0 160 015, which is hereby incorporated by reference. Mixing 75 g of preparation with 1 litre of water produces a suspension or a solution of the preparation suitable for administration to calves.

## Claims

1. A preparation comprising:
5-30% by weight of Isphagula Husk, and
1-20% by weight of at least one amino acid, and
20-80% by weight of at least one carbohydrate and electrolytes for use as a therapeutical agent.

2. Use of a preparation comprising:
5-30% by weight of Isphagula Husk,
1-20% by weight of at least one amino acid, and
20-80% by weight of at least one carbohydrate and electrolytes for the preparation of a medicament for simultaneous or sequential use in treating a state of disorder on the intestinal system of monogastric animals, including human beings.

3. Use of a preparation comprising:
5-30% by weight of Isphagula Husk,
1-20% by weight of at least one amino acid, and
20-80% by weight of at least one carbohydrate and electrolytes for the preparation of a medicament for treating a state of disorder on the intestinal system of monogastric animals, including human beings.

4. Use of a preparation comprising:
5-30% by weight of Isphagula Husk,
1-20% by weight of at least one amino acid, and
20-80% by weight of at least one carbohydrate and electrolytes for the preparation of a medicament for restoring the epithelium layer of the intestines of mammals, including human beings.

5. A preparation or use according to any one of claims 1 to 4, wherein the amount of Isphagula Husk is in the interval of 10-30% by weight, preferably 15-30% by weight, more preferably 25-30% by weight.

6. A preparation or use according to any one of claims 1 to 4, wherein the amount of the at least one amino acid is in the interval of 1-12% by weight, preferably 2-9 % by weight, more preferably 3-7 % by weight.

7. A preparation or use according to any one of claims 1 to 4, wherein the amount of carbohydrate is in the interval of 25-50% by weight, preferably 30-45% by weight, more preferably 35-40% by weight.

8. A preparation or use according to any one of claims 1 to 4, wherein the amount of electrolyte is in the interval of 8-40% by weight, preferably 12-30% by weight, more preferably 15-25% by weight.

9. A preparation or use according to any one of the preceding claims, wherein the at least one amino acid is comprised in the soluble components of lactic yeast.

10. A preparation or use according to any one of the preceding claims, comprising at least one amino acid selected from the group consisting of all known amino acids, preferably at least one amino acid selected from the group consisting of glutamine, arginine, lysine, histidine, phenylalanine, tyrosine, leucine, isoleucine, methionine, valine, alanine, glycine, proline, glutamic acid, serine, threonine, aspartic acid, tryptophan, cystine, more preferably at least one amino acid selected from the group consisting of glutamine, arginine, alanine and glycine.

11. A preparation or use according to any one of the preceding claims, wherein the amount of glutamine is in the interval of up to 10% by weight, preferably up to 5% by weight, more preferably 0.1-4% by weight, even more preferably 0.2-3% by weight.

12. A preparation or use according to any one of the preceding claims, wherein the amount of arginine is in the interval of up to 5% by weight, preferably up to 3% by weight, more preferably 0.1-2% by weight, even more preferably 0.1-0.5% by weight.

13. A preparation or use according to any one of the preceding claims, wherein at least one of the salts comprised by the electrolytes and is at least one of the salts which will replace at least one of the salts lost by diarrhoea.

14. A preparation or use according to any one of the preceding claims, wherein said at least one carbohydrate is glucose.

15. A preparation or use according to any one of the preceding claims, wherein the electrolytes are a mixture of at least two of the substances selected from the group consisting of magnesium oxide, magnesium carbonate hydroxide, magnesium hydroxide, magnesium silicate, calcium silicate, calcium carbonate, sodium chloride, potassium chloride, sodium hydrogen carbonate, potassium hydrogen carbonate, aluminium phosphate, aluminium hydroxide, citric acid, sodium citrate, trisodium citrate dihydrate and potassium citrate.

16. A preparation or use according to any one of the preceding claims, wherein the electrolytes are a mixture of at least two of the substances selected from the group consisting of magnesium hydroxide, sodium chloride, potassium chloride, sodium hydrogen carbonate, citric acid, trisodium citrate dihydrate and sodium citrate.

17. A preparation or use according to any one of the preceding claims, further comprising at least one filler, at least one taste corrigent, at least one colouring agent.

18. A preparation or use according to any one of the preceding claims, further comprising a filler.

19. A preparation or use according to claim 18, wherein the filler is a fibrous bran material.

20. A preparation or use according to claim 18, wherein the filler is wheat flour.

21. A preparation or use according to any one of the preceding claims, further comprising a pharmaceutically acceptable colouring agent.

22. A preparation or use according to any one of the preceding claims, wherein the colouring agent is FD&C RED #40.

23. A preparation or use according to any one of the preceding claims, further comprising alfa-tocoferol (natural vitamin E).

24. A preparation or use according to any one of the preceding claims, wherein said preparation consists of 27.16% Isphagula Husk,10.66% of lactic yeast mixture including glutamine, 19.75% electrolytes which are made up of 3.30% potassium chloride, 7.08% sodium hydrogen carbonate, 4.85% sodium chloride, 3.45% trisodium citrate dihydrate, 1.07% magnesium hydroxide; 38.10% dextrose monohydrate, 0.87% nicotinamide, 0.30% flavouring agent, 0.20% silicium dioxide, 2.43% wheat flour, 0.03% feed colouring agent, 0.50% alfa-tocoferol (natural vitamin E), where the percent by weight is calculated on the basis of the finished preparation.

25. Use of a preparation according to any one of the preceding claims for the manufacture of a medicament for treating diarrhoea.

## Patentansprüche

1. Zubereitung, welche enthält:
5-30 Gew.-% Isphagula Husk, und
1-20 Gew.-% mindestens einer Aminosäure und
20-80 Gew.-% mindestens eines Kohlenhydrats und Elektrolyte; für die Verwendung als therapeutisches Mittel.

2. Verwendung einer Zubereitung, welche enthält:
5-30 Gew.-% Isphagula Husk, und
1-20 Gew.-% mindestens einer Aminosäure und
20-80 Gew.-% mindestens eines Kohlenhydrats und Elektrolyte; für die Herstellung eines Arzneimittels für die gleichzeitige oder aufeinander folgende Verwendung in der Behandlung eines Störungszustandes des Verdauungssystems von monogastrischen Tieren, einschließlich Menschen.

3. Verwendung einer Zubereitung, welche enthält:
5-30 Gew.-% Isphagula Husk, und
1-20 Gew.-% mindestens einer Aminosäure und
20-80 Gew.-% mindestens eines Kohlenhydrats und Elektrolyte; für die Herstellung eines Arzneimittels zur Behandlung eines Störungszustandes des Verdauungssystems von monogastrischen Tieren, einschließlich Menschen.

4. Verwendung einer Zubereitung, welche enthält:
5-30 Gew.-% Isphagula Husk, und
1-20 Gew.-% mindestens einer Aminosäure und
20-80 Gew.-% mindestens eines Kohlenhydrats und Elektrolyte; für die Zubereitung eines Arzneimittels zur Wiederherstellung der Epithelschicht des Verdauungssystems von Säugern, einschließlich Menschen.

5. Verwendung nach einem der Ansprüche 1 bis 4, wobei die Menge von Isphagula Husk zwischen 10 und 30 Gew.-%, vorzugsweise 15 bis 30 Gew.-%, stärker bevorzugt 25 bis 30 Gew.-% ist.

6. Zubereitung oder Verwendung nach einem der Ansprüche 1 bis 4, wobei die Menge der mindestens einen Aminosäure zwischen 1 und 12 Gew.-%, vorzugsweise 2 bis 9 Gew.-%, stärker bevorzugt 3 bis 7 Gew.-% ist.

7. Zubereitung oder Verwendung nach einem der Ansprüche 1 bis 4, wobei die Menge des Kohlenhydrats zwischen 25 und 50 Gew.-%, vorzugsweise 30 bis 45 Gew.-%, stärker bevorzugt 35 bis 40 Gew.-% ist.

8. Zubereitung oder Verwendung nach einem der Ansprüche 1 bis 4, wobei die Menge des Elektrolyten zwischen 8 und 40 Gew.-%, vorzugsweise 12 bis 30 Gew.-%, stärker bevorzugt 15 bis 25 Gew.-% ist.

9. Zubereitung oder Verwendung nach einem der vorstehenden Ansprüche, wobei die mindestens eine Aminosäure in den löslichen Komponenten von Milchhefe enthalten ist.

10. Zubereitung oder Verwendung nach einem der vorstehenden Ansprüche, die mindestens eine Aminosäure umfasst, die aus der Gruppe ausgewählt ist, die aus allen bekannten Aminosäuren besteht, vorzugsweise mindestens eine Aminosäure, die aus der Gruppe ausgewählt ist, die aus Glutamin, Arginin, Lysin, Histidin, Phenylalanin, Tyrosin, Leucin, Isoleucin, Methionin, Valin, Alanin, Glycin, Prolin, Glutaminsäure, Serin, Threonin, Asparaginsäure, Tryptophan und Cystein besteht, stärker bevorzugt mindestens eine Aminosäure, die aus der Gruppe ausgewählt ist, die aus Glutamin, Arginin, Alanin und Glycin besteht.

11. Zubereitung oder Verwendung nach einem der vorstehenden Ansprüche, wobei die Menge des Glutamins bis zu 10 Gew.-%, vorzugsweise bis zu 5 Gew.-%, stärker bevorzugt 0,1 bis 4 Gew.-% und noch stärker bevorzugt 0,2 bis 3 Gew.-% ist.

12. Zubereitung oder Verwendung nach einem der vorstehenden Ansprüche, wobei die Menge des Arginins bis zu 5 Gew.-%, vorzugsweise bis zu 3 Gew.-%, stärker bevorzugt 0,1 bis 2 Gew.-% und noch stärker bevorzugt 0,1 bis 0,5 Gew.-% ist.

13. Zubereitung oder Verwendung nach einem der vorstehenden Ansprüche, wobei mindestens eines der von den Elektrolyten umfassten Salze mindestens eines der Salze ist, welche mindestens eines der durch Durchfall verlorengehenden Salze ersetzt.

14. Zubereitung oder Verwendung nach einem der vorstehenden Ansprüche, wobei das mindestens eine Kohlenhydrat Glucose ist.

15. Zubereitung oder Verwendung nach einem der vorstehenden Ansprüche, wobei die Elektrolyten ein Gemisch von mindestens 2 der Substanzen sind, die aus der Gruppe ausgewählt sind, die aus Magnesiumoxid, Magnesiumcarbonathydroxid, Magnesiumhydroxid, Magnesiumsilicat, Calciumsilicat, Calciumcarbonat, Natriumchlorid, Kaliumchlorid, Natriumhydrogencarbonat, Kaliumhydrogencarbonat, Aluminiumphosphat, Aluminiumhydroxid, Citronensäure, Natriumcitrat, Trinatriumcitratdihydrat und Kaliumcitrat besteht.

16. Zubereitung oder Verwendung nach einem der vorstehenden Ansprüche, wobei die Elektrolyten ein Gemisch von mindestens 2 der Substanzen sind, die aus der Gruppe ausgewählt sind, die aus Magenesiumhydroxid, Natriumchlorid, Kaliumchlorid, Natriumhydrogencarbonat, Citronensäure, Trinatriumcitratdihydrat und Natriumcitrat besteht.

17. Zubereitung oder Verwendung nach einem der vorstehenden Ansprüche, die außerdem mindestens einen Füllstoff, mindestens ein Geschmackskorrigent und mindestens ein Farbmittel umfasst.

18. Zubereitung oder Verwendung nach einem der vorstehenden Ansprüche, die außerdem einen Füllstoff umfasst.

19. Zubereitung oder Verwendung nach Anspruch 18, wobei der Füllstoff ein faseriges Kleiematerial ist.

20. Zubereitung oder Verwendung nach Anspruch 18, wobei der Füllstoff Weizenmehl ist.

21. Zubereitung oder Verwendung nach einem der vorstehenden Ansprüche, die außerdem ein pharmazeutisch geeignetes Farbmittel umfasst.

22. Zubereitung oder Verwendung nach einem der vorstehenden Ansprüche, wobei das Färbemittel FD&C RED #40 ist.

23. Zubereitung oder Verwendung nach einem der vorstehenden Ansprüche, die außerdem alfa-Tocoferol (natürliches Vitamin E) umfasst.

24. Zubereitung oder Verwendung nach einem der vorstehenden Ansprüche, wobei die Zubereitung aus 27,16 % Isphagula Husk, 10,66 % Glutamin einschließendes Milchhefegemisch, 19,75 % Elektrolyten, zusammengesetzt aus 3,30 % Kaliumchlorid, 7,08 % Natriumhydrogencarbonat, 4,85 % Natriumchlorid, 3,45 % Trinatriumcitratdihydrat und 1,07 % Magnesiumhydroxid, 38,10 % Dextrosemonohydrat, 0,87 % Nicotinamid, 0,30 % Aromamittel, 0,20 % Siliciumdioxid, 2,43 % Weizenmehl, 0,03 % Futterfarbmittel und 0,50 % alfa-Tocoferol (natürliches Vitamin E) besteht, wobei die Gewichtsprozente auf Basis der fertigen Zubereitung berechnet sind.

25. Verwendung einer Zubereitung nach einem der vorstehenden Ansprüche für die Herstellung eines Arzneimittels zur Behandlung von Durchfall.

## Revendications

1. Composition comprenant :
5 à 30 % en poids de cosse d'Ispaghula Husk, et
1 à 20 % en poids d'au moins un acide aminé, et
20 à 80 % en poids d'au moins un glucide et des électrolytes utilisables comme agent thérapeutique.

2. Utilisation d'une préparation comprenant :
5 à 30 % en poids de cosse d'Ispaghula Husk,
1 à 20 % en poids d'au moins un acide aminé, et
20 à 80 % en poids d'au moins un glucide et des électrolytes pour la préparation d'un médicament utilisable simultanément ou séquentiellement dans le traitement d'un état de trouble sur le système intestinal d'animaux monogastriques, dont les êtres humains.

3. Utilisation d'une préparation comprenant :
5 à 30 % en poids de cosse d'Ispaghula Husk,
1 à 20 % en poids d'au moins un acide aminé, et
20 à 80 % en poids d'au moins un glucide et des électrolytes pour la préparation d'un médicament destiné à traiter un état de trouble sur le système intestinal d'animaux monogastriques, dont les êtres humains.

4. Utilisation d'une préparation comprenant :
5 à 30 % en poids de cosse d'Ispaghula Husk,
1 à 20 % en poids d'au moins un acide aminé, et
20 à 80 % en poids d'au moins un glucide et des électrolytes pour la préparation d'un médicament destiné à restaurer la couche épithéliale des intestins de mammifères, dont les être humains.

5. Préparation ou utilisation selon l'une quelconque des revendications 1 à 4, dans laquelle la quantité de cosse d'Ispaghula Husk se trouve dans l'intervalle de 10 à 30 % en poids, de préférence de 15 à 30 % en poids, et de manière davantage préférée de 25 à 30 % en poids.

6. Préparation ou utilisation selon l'une quelconque des revendications 1 à 4, dans laquelle la quantité du au moins un acide aminé se trouve dans l'intervalle de 1 à 12 % en poids, de préférence de 2 à 9 % en poids, et de manière davantage préférée de 3 à 7 % en poids.

7. Préparation ou utilisation selon l'une quelconque des revendications 1 à 4, dans laquelle la quantité de glucide se trouve dans l'intervalle de 25 à 50 % en poids, de préférence de 30 à 45 % en poids, et de manière davantage préférée de 35 à 40 % en poids.

8. Préparation ou utilisation selon l'une quelconque des revendications 1 à 4, dans laquelle la quantité d'électrolyte se trouve dans l'intervalle de 8 à 40 % en poids, de préférence de 12 à 30 % en poids, et de manière davantage préférée de 15 à 25 % en poids.

9. Préparation ou utilisation selon l'une quelconque des revendications précédentes, dans laquelle le au moins un acide aminé est compris dans les composants solubles de la levure lactique.

10. Préparation ou utilisation selon l'une quelconque des revendications précédentes, comprenant au moins un acide aminé choisi dans le groupe constitué de tous les acides aminés connus, de préférence au moins un acide aminé choisi dans le groupe constitué par la glutamine, l'arginine, la lysine, l'histidine, la phénylalanine, la tyrosine, la leucine, l'isoleucine, la méthionine, la valine, l'alanine, la glycine, la proline, l'acide glutamique, la sérine, la thréonine, l'acide aspartique, le tryptophane, la cystine, et de manière davantage préférée au moins un acide aminé choisi dans le groupe constitué par la glutamine, l'arginine, l'alanine et la glycine.

11. Préparation ou utilisation selon l'une quelconque des revendications précédentes, dans laquelle la quantité de glutamine se trouve dans un intervalle allant jusqu'à 10 % en poids, de préférence jusqu'à 5 % en poids, et de manière davantage préférée de 0,1 à 4 % en poids, ou de manière encore davantage préférée de 0,2 à 3 % en poids.

12. Préparation ou utilisation selon l'une quelconque des revendications précédentes, dans laquelle la quantité d'arginine se trouve dans un intervalle allant jusqu'à 5 % en poids, de préférence jusqu'à 3 % en poids, et de manière davantage préférée de 0, 1 à 2 % en poids, ou de manière encore davantage préférée de 0,1 à 0,5 % en poids.

13. Préparation ou utilisation selon l'une quelconque des revendications précédentes, dans laquelle au moins un des sels est compris par les électrolytes et est au moins un des sels qui remplaceront au moins un des sels perdus par diarrhée.

14. Préparation ou utilisation selon l'une quelconque des revendications précédentes, dans laquelle ledit au moins un glucide est le glucose.

15. Préparation ou utilisation selon l'une quelconque des revendications précédentes, dans laquelle les électrolytes sont un mélange d'au moins deux des substances choisies dans le groupe constitué par l'oxyde de magnésium, l'hydroxyde de carbonate de magnésium, l'hydroxyde de magnésium, le silicate de magnésium, le silicate de calcium, le carbonate de calcium, le chlorure de sodium, le chlorure de potassium, l'hydrogénocarbonate de sodium, l'hydrogénocarbonate de potassium, le phosphate d'aluminium, l'hydroxyde d'aluminium, l'acide citrique, le citrate de sodium, le citrate trisodique dihydraté et le citrate de potassium.

16. Préparation ou utilisation selon l'une quelconque des revendications précédentes, dans laquelle les électrolytes sont un mélange d'au moins deux des substances choisies dans le groupe constitué par l'hydroxyde de magnésium, le chlorure de sodium, le chlorure de potassium, l'hydrogénocarbonate de sodium, l'acide citrique, le citrate trisodique dihydraté et le citrate de sodium.

17. Préparation ou utilisation selon l'une quelconque des revendications précédentes, comprenant en outre au moins une matière de remplissage, au moins un correcteur de goût, au moins un agent colorant.

18. Préparation ou utilisation selon l'une quelconque des revendications précédentes, comprenant en outre une matière de remplissage.

19. Préparation ou utilisation selon la revendication 18, dans laquelle la matière de remplissage est une matière fibreuse de son.

20. Préparation ou utilisation selon la revendication 18, dans laquelle la matière de remplissage est la farine de froment.

21. Préparation ou utilisation selon l'une quelconque des revendications précédentes, comprenant en outre un agent colorant pharmaceutiquement acceptable.

22. Préparation ou utilisation selon l'une quelconque des revendications précédentes, dans laquelle l'agent colorant est le FD&C RED #40.

23. Préparation ou utilisation selon l'une quelconque des revendications précédentes, comprenant en outre de l'alfa-tocophérol (vitamine E naturelle).

24. Préparation ou utilisation selon l'une quelconque des revendications précédentes, dans laquelle ladite préparation est composée de 27,16 % de cosse d'Ispaghula Husk, 10,66 % d'un mélange de levure lactique comprenant de la glutamine, 19,75 % d'électrolytes qui sont composés de 3,30 % de chlorure de potassium, 7,08 % d'hydrogénocarbonate de sodium, 4,85 % de chlorure de sodium, 3,45 % de citrate trisodique dihydraté, 1,07 % d'hydroxyde de magnésium ; 38,10 % de monohydrate de dextrose, 0,87 % de nicotinamide, 0,30 % d'aromatisant, 0,20 % de dioxyde de silicium, 2,43 % de farine de froment, 0,03 % d'agent colorant alimentaire, 0,50 % d'alfa-tocophérol (vitamine E naturelle), où le pourcentage en poids est calculé en se basant sur la préparation finie.

25. Utilisation d'une préparation selon l'une quelconque des revendications précédentes pour la fabrication d'un médicament destiné à traiter les diarrhées.
